# NEW EUROPEAN PATENT SPECIFICATION

(11) **EP 3 773 228 B2**
(45) Date of publication and mention of the opposition decision: **25.11.2026**
(45) Mention of the grant of the patent: 21.02.2024
(21) Application number: 19784813.8
(22) Date of filing: 10.03.2019
(51) Int. Cl.: B06B 1/02, B06B 1/06, G01H 11/08, G01S 7/52, G01S 15/89

(54) **IMAGING DEVICES HAVING PIEZOELECTRIC TRANSCEIVERS**
BILDGEBUNGSVORRICHTUNGEN MIT PIEZOELEKTRISCHEN SENDEEMPFÄNGERN
DISPOSITIFS D'IMAGERIE POURVU D'ÉMETTEURS-RÉCEPTEURS PIÉZOÉLECTRIQUES

(30) Priority: 11.04.2018 US 201815951118
(43) Date of publication of application: 17.02.2021
(60) Divisional application: 24158305.3 / 4 345 502
(73) Proprietor: Exo Imaging Inc., Redwood City CA 94065 (US)
(72) Inventor: AKKARAJU, Sandeep, Wellesley, Massachusetts 02482 (US); KWON, Haesung, Austin, Texas 78750 (US); BIRCUMSHAW, Brian, Oakland, California 94618 (US)
(74) Representative: Viering, Jentschura & Partner mbB Patent- und Rechtsanwälte
(86) International application number: PCT/US2019/021515
(87) International publication number: WO 2019/199398

(56) References cited:
- WO-A1-2017/216139
- WO-A1-2017/216139
- US-A1- 2007 103 697
- US-A1- 2008 009 741
- US-A1- 2008 009 741
- US-A1- 2012 289 897
- US-A1- 2013 293 065
- US-A1- 2013 293 065
- Mukhiya, Ravindra, "Fabrication of Capacitive Micromachined Ultrasonic Transducer Arrays With Isolation Trenches Using Anodic Wafer Bonding" IEEE SENSORS JOURNAL, Vol. 15, No.9. September 2015

## Description

### BACKGROUND

### A. Technical Field

The present invention relates to imaging devices, and more particularly, to imaging devices having micromachined ultrasound transducers (MUTs).

### B. Background of the Invention

A non-intrusive imaging system for imaging internal organs of a human body and displaying images of the internal organs transmits signals into the human body and receives signals reflected from the organ. Typically, transducers that are used in an imaging system are referred to as transceivers and some of the transceivers are based on photo-acoustic or ultrasonic effects. In general, transceivers are used for imaging as well as other applications, such as medical imaging, flow measurements in pipes, speaker, microphone, lithotripsy, heating tissue for therapeutics, and highly intensive focused ultrasound (HIFU) for surgery.

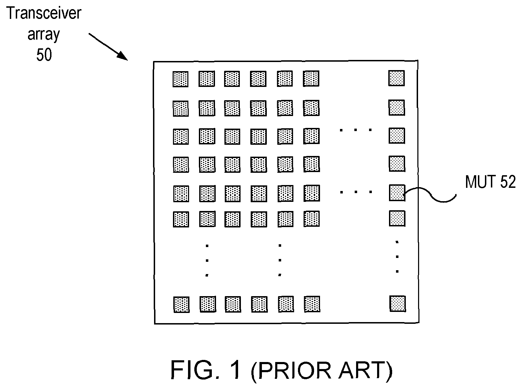

Advances in micro-machining technologies allow sensors and actuators to be efficiently incorporated on a substrate. In particular, micromachined ultrasound transducers (MUTs), using capacitive transducers (cMUTs) or piezoelectric transducers (pMUTs), are particularly advantageous compared to the conventional MUTs having a large form factor. FIG. 1 shows a two dimensional rectilinear transceiver array **50** in a conventional system. As depicted, the transceiver array **50** may include: a set of MUTs **52** that generates and transmit pressure waves in a transmit mode/process and that receive pressure waves and develop electrical charge in response to the received pressure waves in a receive mode/process. As depicted, the MUTs **52** are uniformly spaced in the x- and y-directions, i.e., the distance from one MUT to a neighboring MUT along the x-direction (or the y-direction) is the same throughout the array. The MUTs **52** tend to have a limited number of vibrational resonances, i.e., the MUTs array **52** may have a limited bandwidth in the frequency domain. In general, the wider bandwidth the MUTs **52** have, the more sophisticated operational modes the MUTs **52** may be operated in and the better images the transceiver array **50** may be able to generate. As such, there is a strong need to design MUTs that have increased bandwidth for enhanced acoustic performance.

US 2013/293065 A1 discloses a transducer array including a plurality of micromachined ultrasonic transducers in a staggered arrangement, wherein each MUT of the plurality of MUTs has a dimension along a first direction that is larger than a dimension along a second direction.

### Summary of the disclosure

In order to solve the deficiencies of prior art a transducer array is provided according to claim 1. Specific advantageous embodiments are provided according to the dependent claims.

In exemplary embodiments, an array of micromachined ultrasonic transducers (MUTs) includes MUTs arranged in a two dimensional array that has first and second rows, the MUTs in the first row being equally spaced by a horizontal pitch in a horizontal direction, the MUTs in the second row being equally spaced by the horizontal pitch in the horizontal direction. The MUTs in the second row are shifted along the horizontal direction by a first horizontal distance relative to the MUTs in the first row and shifted along a vertical direction by a first vertical distance relative to the MUTs in the first row. The first horizontal distance is greater than zero and less than the horizontal pitch. The first vertical distance ranges from one tenth of a horizontal width of a MUT of the plurality of MUTs to a half of a vertical height of the MUT.

In embodiments, an imaging system includes a transceiver tile for generating a pressure wave and converting an external pressure wave into an electrical signal, and a control unit for controlling the transceiver tile. The transceiver tile includes an array of micromachined ultrasonic transducers (MUTs), where the array includes MUTs arranged in a two dimensional array that has first and second rows, the MUTs in the first row being equally spaced by a horizontal pitch in a horizontal direction, the MUTs in the second row being equally spaced by the horizontal pitch in the horizontal direction. The MUTs in the second row are shifted along the horizontal direction by a first horizontal distance relative to the MUTs in the first row and shifted along a vertical direction by a first vertical distance relative to the MUTs in the first row. The first horizontal distance is greater than zero and less than the horizontal pitch. The first vertical distance ranges from one tenth of a horizontal width of a MUT of the plurality of MUTs to a half of a vertical height of the MUT.

### Brief Description of the Drawings

References will be made to embodiments of the invention, examples of which may be illustrated in the accompanying figures. These figures are intended to be illustrative, not limiting. Although the invention is generally described in the context of these embodiments, it should be understood that it is not intended to limit the scope of the invention to these particular embodiments.
Figure (or "FIG.") 1 shows a two dimensional rectilinear transceiver array in a conventional system.
FIG. 2 shows an imaging system according to embodiments of the present disclosure.
FIG. 3 shows a schematic diagram of an imager according to embodiments of the present disclosure.
FIG. 4 shows an asymmetric (or staggered) rectilinear transceiver array according to embodiments of the present disclosure.
FIG. 5 shows an enlarged view of a MUT array according to embodiments of the present disclosure.
FIG. 6 shows an enlarged view of a MUT array according to embodiments of the present disclosure.
FIG. 7 shows two pairs of rectangular MUTs and pads that are arranged in a staggered configuration according to embodiments of the present disclosure.
FIGS. 8A - 8D show a set of vibrational mode shapes of a MUT, taken along a direction 7-7 in FIG. 7, according to embodiments of the present disclosure.
FIG. 9 shows a frequency response plot of an asymmetrical array according to embodiments of the present disclosure.
FIG. 10 shows a plot of acoustic beam pattern responses of MUT arrays as functions of elevation angle according to embodiments of the present disclosure.
FIG. 11 shows two pairs of elliptical MUTs and pads that are arranged in a staggered configuration according to embodiments of the present disclosure.
FIG. 12 shows an asymmetric (or staggered) rectilinear transceiver array according to embodiments of the present disclosure.
FIGS. 13A and 13B show top and cross-sectional views of an exemplary MUT according to embodiments of the present disclosure.

### Detailed Description of the Preferred Embodiments

In the following description, for purposes of explanation, specific details are set forth in order to provide an understanding of the disclosure. It will be apparent, however, to one skilled in the art that the disclosure can be practiced without these details. Furthermore, one skilled in the art will recognize that embodiments of the present disclosure, described below, may be implemented in a variety of ways, such as a process, an apparatus, a system, or a device, without departing from the scope of the appended claims.

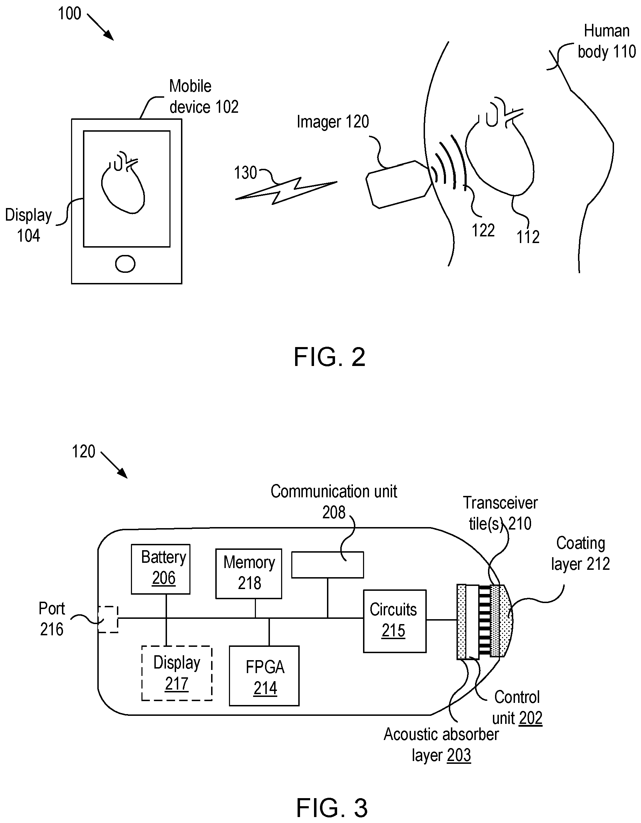

FIG. 2 shows an imaging system 100 according to embodiments of the present disclosure. As depicted, the system 100 may include: an imager 120 that generates and transmit pressure waves **122** toward an internal organ **112,** such as heart, in a transmit mode/process; and a device **102** that communicates signals to the imager through a communication channel **130.** In embodiments, the internal organ **112** may reflect a portion of the pressure waves **122** toward the imager **120,** and the imager **120** may capture the reflected pressure waves and generate electrical signals in a receive mode/process. The imager **120** may communicate the electrical signals to the device **102** and the device **102** may display images of the human organ on a display/screen **104** using the electrical signals.

It is noted that the imager **120** may be used to get an image of internal organs of an animal, too. It is also noted that the pressure wave **122** may be acoustic, ultrasonic, or photo-acoustic waves that can travel through the human/animal body and be reflected by the internal organs.

In embodiments, the imager **120** may be a portable device and communicate signals through the communication channel **130**, either wirelessly or via a cable, with the device **102**. In embodiments, the device **102** may be a mobile device, such as cell phone or iPad, or a stationary computing device that can display images to a user.

FIG. 3 shows a schematic diagram of the imager **120** according to embodiments of the present disclosure. In embodiments, the imager **120** may be an ultrasonic imager. As depicted in FIG. 2, the imager **120** may include: a transceiver tile(s) **210** for transmitting and receiving pressure waves; a coating layer **212** that operates as a lens for focusing the pressure waves and also functions as an impedance interface between the transceiver tile and the human body **110;** a control unit **202,** such as ASIC chip, for controlling the transceiver tile(s) **210;** a microprocessor **214** for controlling the components of the imager **120;** a communication unit **208** for communicating data with an external device, such as the device **102,** through one or more ports **230;** a memory **218** for storing data; a battery **206** for providing electrical power to the components of the imager; and optionally a display **216** for displaying images of the target organs.

In embodiments, the device **102** may have a display/screen. In such a case, the display may not be included in the imager **120.** In embodiments, the imager **120** may receive electrical power from the device **102** through one of the ports **230.** In such a case, the imager **120** may not include the battery **206.** It is noted that one or more of the components of the imager **120** may be combined into one integral electrical element. Likewise, each component of the imager **120** may be implemented in one or more electrical elements.

In embodiments, the user may apply gel on the coating layer **212** so that the impedance matching between the coating layer **212** and the human body **110** may be improved, i.e., the power loss at the interface is reduced.

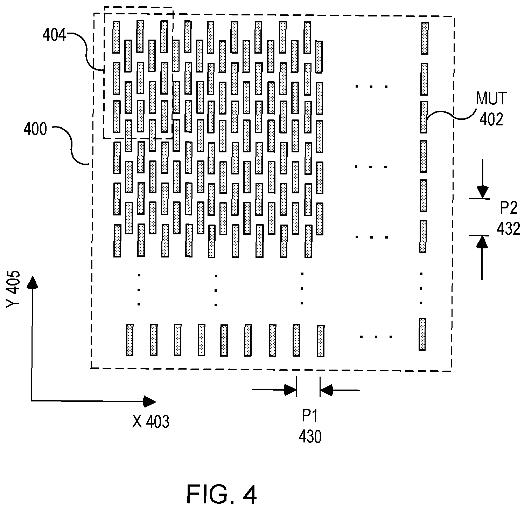

FIG. 4 shows an enlarged view of an asymmetric (or staggered) rectilinear transceiver array **400** including MUTs **402** according to embodiments of the present disclosure. In embodiments, the array **400** may be included in the transceiver tile **210.** The MUTs **402** may be arranged in a staggered configuration to bring a benefit of the array acoustic performance. Hereinafter, the term staggered (or asymmetric) array refers to an array of MUTs, where the MUTs in a first row are shifted along the x-direction **403** relative to the MUTs in a second row. In embodiments, the MUTs in a row may be equally spaced by a horizontal distance (pitch) P1 **430** and the MUTs in a column may be equally spaced by a vertical distance (pitch) P2 **432.**

In embodiments, each MUT **402** may by a pMUT and include a piezoelectric layer formed of at least one of PZT, KNN, PZT-N, PMN-Pt, AlN, Sc-AlN, ZnO, PVDF, and LiNiO₃. In alternative embodiments, each MUT 402 may be a cMUT. In FIG. 4, each MUT **402** is shown to have a rectangular shape. More specifically, each MUT may include a top electrode that has a rectangular projection area. It should be apparent to those of ordinary skill in the art that the top electrode may have other suitable geometrical shape, such as, square, circle, ellipse, oval, so on. For the purpose of illustration, in the drawings of the present application, each MUT is symbolically represented by the geometrical shape of the top electrode.

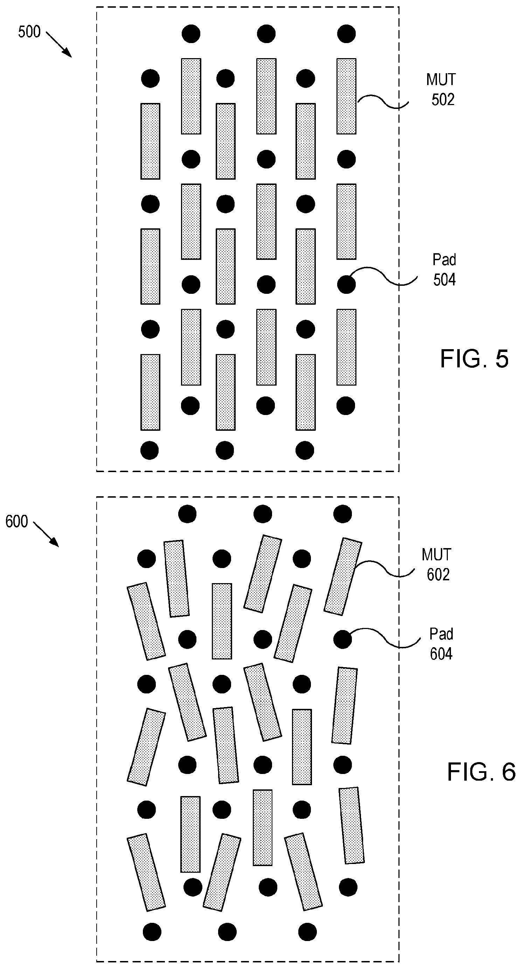

FIG. 5 shows an enlarged view of a MUT array **500** according to embodiments of the present disclosure. In embodiments, the array **500** may correspond to the portion of array **404** in FIG. 4. As depicted, the array **500** may include MUTs **502** and electrical pads **504.** In embodiments, the MUTs **502** and pads **504** are arranged in a staggered configuration. In embodiments, each electrical pad **504** may electrically couple one or more of the MUTs **502** to a substrate, such as an ASIC or an electrical board (not shown in FIG. 5), where the substrate may contain electronics to send/receive electrical signals to/from the MUTs **502.** In embodiments, the substrate may be disposed under the MUT array and each pad **504** may extend in a vertical direction (i.e., in a direction normal to the paper). It is noted that other electrical connections between the MUTs **502** and pads **504** are not shown in FIG. 5, but it should be apparent to those of ordinary skill in the art that suitable electrical connections, such as traces and wires, may be used to electrically connect the MUTs **502** to the pads **504.**

FIG. 6 shows an enlarged view of a MUT array **600** according to embodiments of the present disclosure. In embodiments, the **array 600** may correspond to the portion of array **404** in FIG. 4. In embodiments, the MUTs **602** may be arranged in a staggered or asymmetric configuration and also certain amount of randomness may be introduced to the orientation of each MUT. In embodiments, the randomness may bring acoustic signal enhancement to an imager by reducing the crosstalk among MUTs **602.** In embodiments, the randomly rotated MUT **602** may remain on the x-y plane, while each MUT (602) is substantially aligned with the first direction (Y), and each electrical pad **604** may be disposed in a comparable way to a pad location in the MUT array **500.**

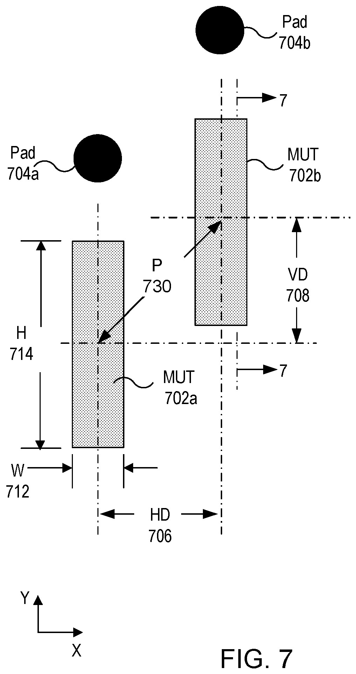

FIG. 7 shows two pairs of MUTs **702** and pads **704** that are arranged in a staggered configuration according to embodiments of the present disclosure. For the purpose of illustration, each MUT is represented by a rectangle that is a projection area of the top electrode of the MUT. In embodiments, the two MUTs **702a** and **702b** in FIG. 7 may be two neighboring MUTs in the MUT array **400.** As depicted, the MUTs **702a** and **702b** may be separated by a horizontal distance, HD, **706** in the x-direction and by a vertical distance, VD, **708** in the y-direction. Each MUT **702** may have a rectangular shape with the width, W, **712** and height, H, **714.** In embodiments, the horizontal distance HD **706** may be greater than zero and less than four times the horizontal width **712** of the MUT and less than the pitch in the x-direction.

Unlike the conventional MUT array **50,** in embodiments, the MUTs **702a** and **702b** may be arranged in a staggered configuration, i.e., the pair of MUT **702a** and pad **704a** in the first row may be shifted along the x-direction relative to the pair of MUT **702b** and pad **704b** in the second row. As a result of the staggered configuration, the MUT array **400** may have one or more asymmetric vibrational modes (or, shortly, asymmetric modes), resulting in a wider bandwidth than the conventional MUT array **50.** As the MUT array **400** may have wider bandwidth, the MUT array may be operated in more sophisticated operational modes.

In general, the number density of the MUTs in a MUT array may affect the resolution of the images generated by the MUT array. In the conventional MUT array **50,** the number density of the MUTs may be increased by decreasing the horizontal distance (or equivalently horizontal pitch) between the MUTs. However, in the conventional MUT array, the mutual impedance between two neighboring MUTs may also increase as the horizontal pitch decreases, which may negate the advantage obtained by the increase of number density. Hereinafter, the mutual impedance refers to the acoustic coupling between two MUTs. In contrast, in embodiments, the diagonal distance, P, **730** may be the effective separation between the MUTs **702a** and **702b.** As such, in embodiments, the mutual impedance may be less than the conventional symmetric MUT array that has the same horizontal separation HD **706.** Stated differently, the staggering configuration may allow the number density to be increased without out increasing mutual impedance significantly.

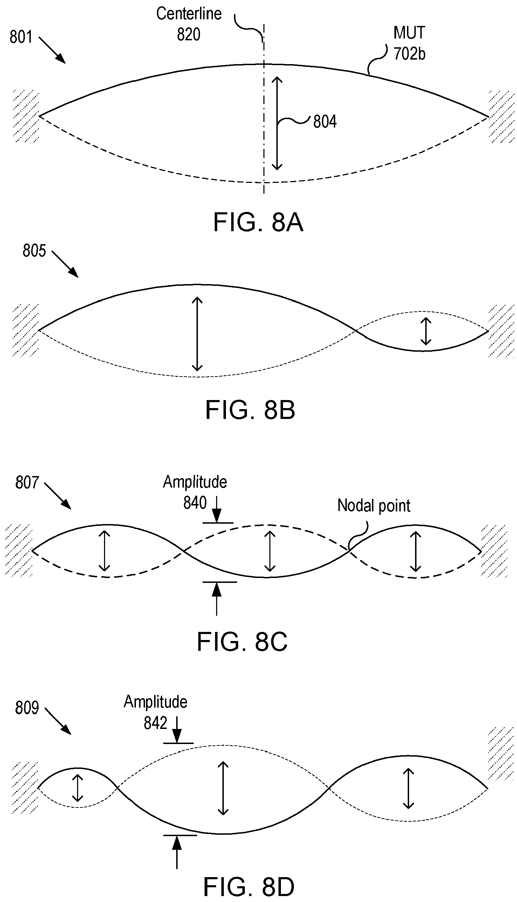

In embodiments, the vertical distance **708** may affect the characteristics of the asymmetric modes of vibration, such as the frequencies of the asymmetric modes and acoustic pressures at vibrational modes, as explained in conjunction with FIGS. 8A - 9. Hereinafter, the term acoustic pressure refers to the level of acoustic power generated by each MUT. In embodiments, the vertical distance **708** may be preferably greater than one tenth of the width **712** and less than the half of the height **714.** In embodiments, as the VD **708** increases up to less than one half of the height **714,** an acoustic pressure amplitude at the asymmetrical modes may increase.

FIGS. 8A - 8D show a set of vibrational mode shapes of the MUT **702b,** taken along the direction 7-7 in FIG. 7, according to embodiments of the present disclosure. For the purpose of illustration, the MUT **702b** is represented by a single line in FIGS. 8A - 8D. However, it should be apparent to those of ordinary skill in the art that the MUT may include a stack of layers. As depicted, FIG. 8A and FIG. 8C present a first vibrational mode 801 and a third vibrational mode **807** of the MUT **702b** at the first and third resonance frequencies respectively, where an arrow **804** indicates the direction of motion of the MUT **702b.** In embodiments, the first and third vibrational modes may be symmetric i e., each mode shape is symmetrical with respect to the centerline **820** of the MUT **720b.** In embodiments, the symmetric modes may be generated even when the MUTs are not staggered, i.e., the symmetric MUT array **50** may have the symmetric vibrational modes in FIGS. 8A and 8C.

In embodiments, FIGS. 8B and 8D show the second vibrational mode **805** and fourth vibrational mode **809** at the second and fourth resonance frequencies of the MUT **702b,** respectively. As depicted, the second and fourth vibrational modes may be asymmetric, i.e., the MUT is not symmetric with respect to the centerline **820.** In embodiments, the asymmetry of the vibrational mode may be obtained by arranging the MUTs in the staggered (asymmetric) configuration, as shown in FIG. 4.

In general, the acoustic pressure performance, which refers to the energy of an acoustic pressure wave generated by each MUT at a frequency, may increase as the peak amplitude of the MUT increases at the frequency. In embodiments, an asymmetric mode may enable wider bandwidth than a symmetric mode when they are vibrating in the same order. For instance, the third mode **807** and fourth mode **809** may have the same order, i.e., the same number of nodal points, and the peak amplitude **840** of the third mode **807** is smaller than the peak amplitude **842** of the fourth mode **809.**

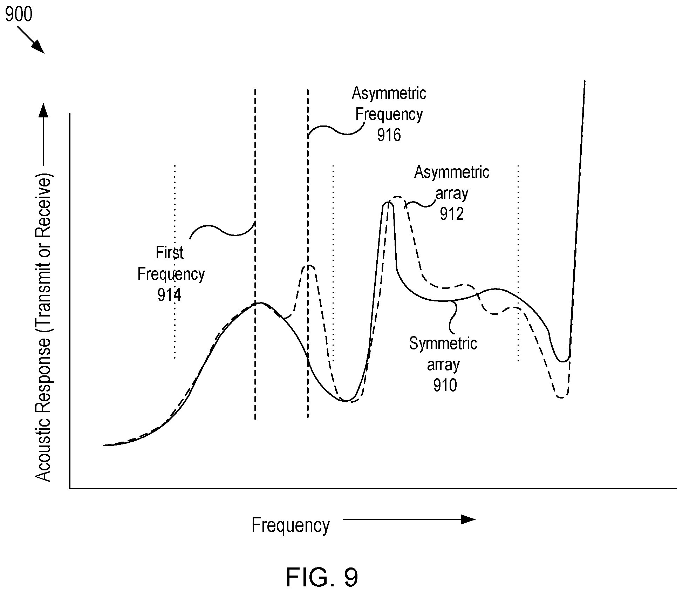

FIG. 9 shows a frequency response plot **900** of the asymmetrical MUT array **400** according to embodiments of the present disclosure. In FIG. 9, the curve **912** shows the response (y-axis) of the array **400** as a function of frequency, where the response refers to the peak amplitude of the pressure waves generated by the array during the transmit mode or the electrical charge developed during the receive mode. In FIG. 9, the curve **910** shows the response of a symmetric array **50** as a function of frequency.

As depicted, the staggered MUT array **400** may have a resonance vibrational mode near the frequency **914**, where the frequency **914** may be also the resonance vibrational mode of the symmetric MUT array **50.** In embodiments, the staggered MUT array **400** in FIG. 7 may have additional resonance frequency at the frequency **916,** which is referred to as asymmetric resonance frequency. In embodiments, with both symmetrical and asymmetrical modes of vibration, as depicted in FIG. 9, the gain of acoustic response may increase, and the bandwidth may improve since the MUT array **400** may be operated at both the center symmetric frequency **914** and a high asymmetric frequency **916.**

It is noted that the frequency response curve **912** may include the contribution from all of the MUTs in the array **400.** Since each MUT in the staggered MUT array **400** may have the same frequency response characteristics, each MUT has the similar frequency response curve to the curve **912,** i.e., the each MUT in the asymmetric array **400** may have resonance frequencies at both the center symmetric frequency **914** and an asymmetric frequency **916.**

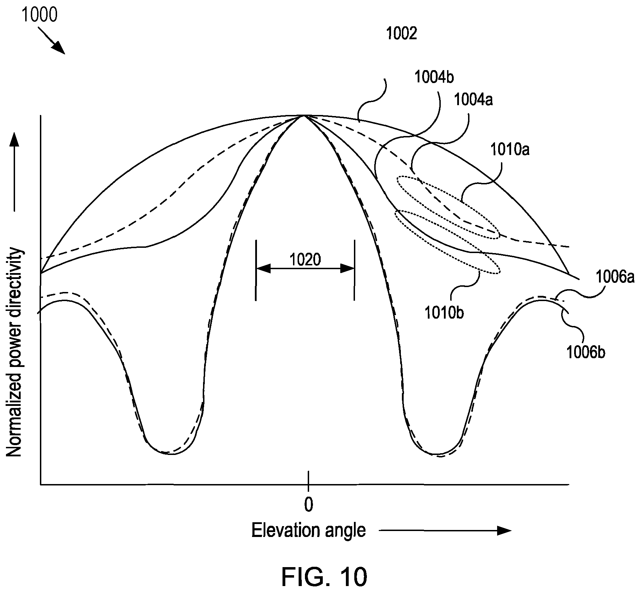

In embodiments, a beamforming technique may be used to direct the pressure waves transmitted by the imager **120** to a particular angle, i.e., the pressure waves from the MUT array **400** may be combined in such a way that pressure waves at a particular angle (i.e., beamforming direction) experience constructive interference while others experience destructive interference. In embodiments, the control unit **202** may control the phase and/or amplitude of the pressure waves generated by the MUT array **400** to steer the beamforming direction. FIG. 10 shows a plot of acoustic beam pattern responses of MUT arrays as functions of elevation angle according to embodiments of the present disclosure. In FIG. 10, each curve shows the acoustic response (y-axis) of a MUT array as a function of elevation angle, where the acoustic response refers to the peak amplitude of the pressure waves generated by the MUT array during the transmit mode (or the electrical charge developed during the receive mode), and the elevation angle refers to the angular distance relative to the beamforming direction.

In general, the directivity, which refers to blocking of noise outside the direction of interest **1020,** affects the signal-to-noise ratio in the beamforming. (Hereinafter, the term direction of interest refers to a preset angular range around the beamforming direction.) In FIG. 10, a curve **1002** may indicate a normalized power directivity for both an a symmetric (staggered) MUT array (such as **400**) and symmetric (non-staggered) array (such as **50**) at the frequency of 1.5 MHz. Curves **1006a** and **1006b** show the directivity of a symmetric and symmetric arrays, respectively, at the frequency of 3.0 MHz. As depicted, the symmetric MUT array may have similar beam patterns as the asymmetric MUT array at the frequencies of 1.5 MHz and 3.0 MHz.

Curves **1004a** and **1004b** show beam patterns of asymmetric and symmetric arrays, respectively, at the frequency of 5.7 MHz. As depicted, the asymmetric array has higher (improved) directivity than the symmetric array, resulting in improved signal-to-noise ratio and image quality. For instance, the curves **1004a** and **1004b** have regions **1010a** and **1010b** that are outside the direction of interest **1020.** As the power level at the region **1010b** is lower than the power level at the region **1010a,** the asymmetric array may have improved signal-to-noise ratio, which in turn improves the image quality.

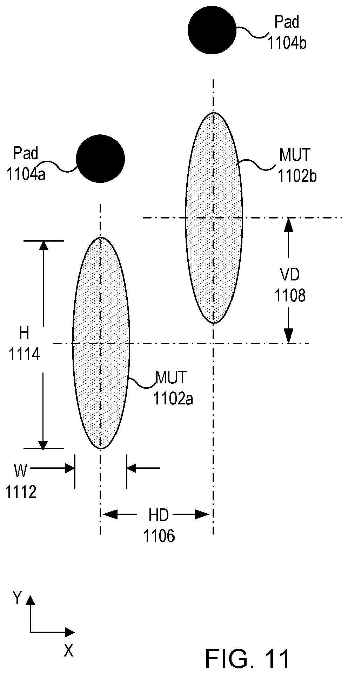

In embodiments, the top electrodes of the MUTs in the array **400** may have different geometrical shapes, such as circle, ellipse, oval, so on. FIG. 11 shows two pairs of elliptical MUTs and circular pads that are arranged in a staggered configuration according to embodiments of the present disclosure. As depicted, the MUTs **1102** may be separated by a horizontal distance, HD, **1106** in the x-direction and by a vertical distance, VD, **1108** in the y-direction. Each MUT **1102** may have an elliptical shape with the width, W, **1112** and height, H, **1114.**

Unlike the conventional MUT array **50,** in embodiments, the MUTs **1102a** and **1102b** may be arranged in a staggered configuration, i.e., the pair of MUT **1102a** and pad **1104a** in the first row may be shifted along the x-direction relative to the pair of MUT **1104b** and pad **1104b** in the second row. In embodiments, the vertical distance **1108** may be preferably greater than one tenth of the horizontal width **1112** and less than one half of the vertical height **1114.** In embodiments, as the VD **1108** increases up to one half of the vertical height **1114,** an acoustic pressure amplitude at the asymmetrical modes may increase.

As a result of the staggered configuration, the MUT array having the staggered configuration in FIG. 11 may have the similar advantages as the MUT array having the staggered configuration in FIG. 7, i.e., the staggered MUT array in FIG. 11 may have improved bandwidth, image resolution, field of view, acoustic pressure, and mutual impedance between MUTs, compared to non-staggered MUT array. In embodiments, the MUTs may be separated by a horizontal distance **1106,** where the horizontal distance **1106** may be greater than zero and less than four times the horizontal width **1112** of the MUT and less than the pitch in the x-direction.

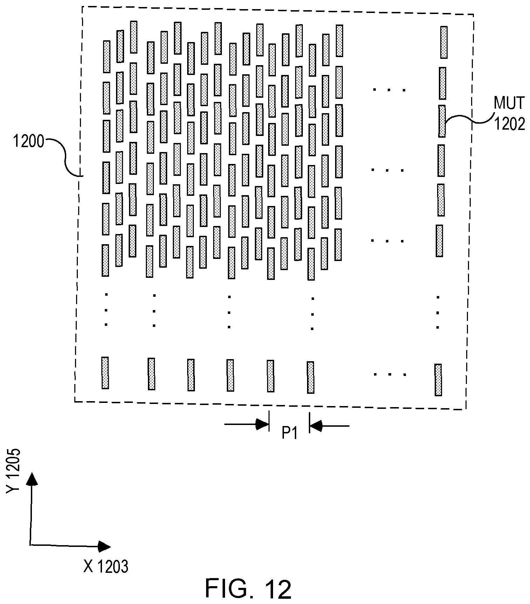

FIG. 12 shows an enlarged view of an asymmetric (or staggered) rectilinear transceiver array **1200** including MUTs **1202** according to embodiments of the present disclosure. The MUTs **1202** may be arranged in a staggered configuration to bring a benefit of the array acoustic performance. As depicted, the MUTs in each row may be equally spaced by a horizontal distance (pitch) and MUTs in each column may be equally spaced by a vertical distance (pitch). In embodiments, the MUTs in the second row may be shifted along the x-direction **1203** relative to the MUTs in the first row by a first horizontal distance, and the MUTs in the third row may be shifted along the x-direction relative to the MUTs in the first row by a second horizontal distance, where the first and second horizontal distances may be greater than zero and less than the horizontal pitch, P1.

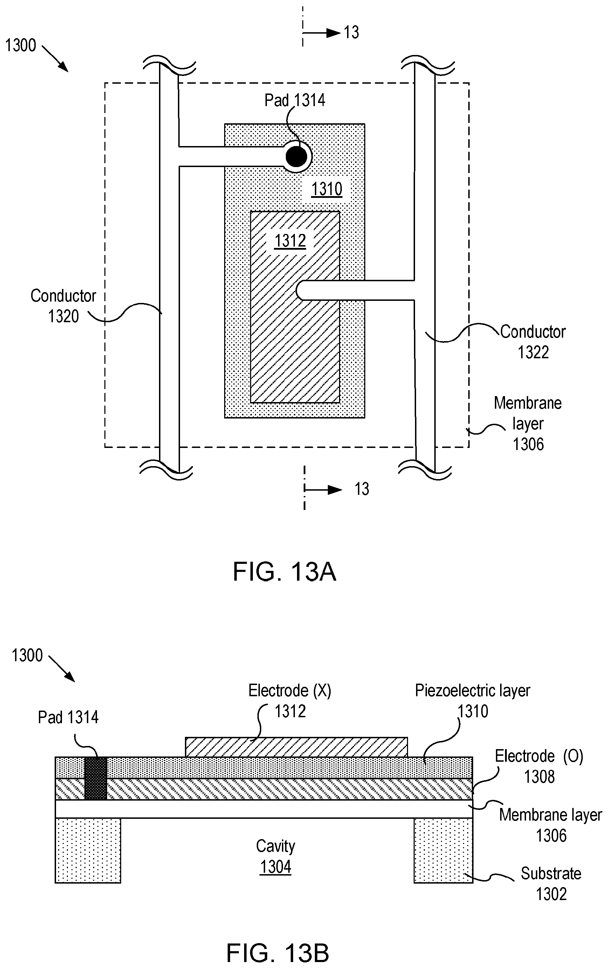

FIG. 13A shows a top view of an exemplary MUT **1300** according to embodiments of the present disclosure. FIG. 13B shows a cross-sectional view of the MUT **1300** in FIG. 13A, taken along the line **13-13,** according to embodiments of the present disclosure. As depicted, the MUT may include: a membrane layer **1306,** suspended from a substrate **1302;** a bottom electrode (O) **1308** disposed on the membrane layer (or, shortly membrane) **1306;** a piezoelectric layer **1310** disposed on the bottom electrode (O) **1308;** and a top electrode (X) **1312** disposed on the piezoelectric layer **1310.** In embodiments, an electrical pad **1314,** which may be a via filled with electrically conducting material, may be formed so that the bottom electrode (O) **1308** may be electrically connected to an electrical conductor **1320.** In embodiments, an electrical conductor **1322** may be electrically connected to the top electrode **1312.** In embodiments, the electrical conductors **1320** and **1322** may be electrical wires or traces formed by patterning a metal layer. In embodiments the pad **1314** and top electrode **1312** may correspond to the pad **704** (or **1104**) and MUT **702** (or **1102**), respectively.

In embodiments, the substrate **1302** and the membrane **1306** may be one monolithic body and the cavity **1304** may be formed to define the membrane **1306.** In embodiments, the cavity **1304** may be filled with a gas at a predetermined pressure or an acoustic damping material to control the vibration of the membrane **1306.**

It is noted that the MUTs in the transceiver arrays **400** and **1200** may have other configuration than the MUT **1300** in FIG. 13A. For instance, each MUT may have more than one top electrode. It should be apparent to those of ordinary skill in the art that each rectangle (or ellipse) in FIGS. 4-7 (or FIGS. 11-12) symbolically represents a MUT, where the MUT may have one or more top electrodes and the top electrodes may have suitable geometrical shapes, such as circle, rectangle, ellipse, so on.

While the invention is susceptible to various modifications and alternative forms, specific examples thereof have been shown in the drawings and are herein described in detail. It should be understood, however, that the invention is not to be limited to the particular forms disclosed, but to the contrary, the invention is to cover all modifications, equivalents, and alternatives falling within the scope of the appended claims.

## Claims

1. A transducer array, comprising:
a plurality of micromachined ultrasonic transducers (MUTs) (402, 502, 602, 702, 1102) in a staggered arrangement, wherein each MUT of the plurality of MUTs has a dimension (714, 1114) along a first direction (Y) that is larger than a dimension (712, 1112) along a second direction (X); and
wherein the staggered arrangement comprises a first row of MUTs aligned along the first direction (Y) and a second row of MUTs aligned along the first direction; the second row being shifted relative to the first row to give an offset distance (VD) (708, 1108) along the first direction between an MUT in the first row (702a, 1102a) and its nearest neighbour in the second row (702b, 1102b);
**characterized in that** a projection area of each of the plurality of MUTs has a shape of rectangle (702) or ellipse (1102).

2. The transducer array of claim 1, wherein:
a center to center distance between the plurality of MUTs in one direction is different from a center to center distance between the plurality of MUTs in another direction.

3. The transducer array of claim 1,
wherein each MUT of the plurality of MUTs has an elongated shape that supports one or more modes of vibration in a flexural mode of operation.

4. The transducer array according to any of claims 1 to 3,
wherein each MUT (602) of the plurality of the MUTs is rotated by a random angle with respect to an axial direction, while each MUT (602) is substantially aligned with the first direction (Y).

5. The transducer array of claim 1,
wherein each of the plurality of MUTs performs at least one of transmitting an ultrasonic wave and receiving an ultrasonic wave.

6. The transducer array of claim 1,
wherein each of the plurality of MUTs operates in at least one of symmetric vibrational mode and asymmetric vibration mode.

## Patentansprüche

1. Eine Wandleranordnung, umfassend:
eine Mehrzahl von mikrobearbeiteten Ultraschallwandlern (MUTs) (402, 502, 602, 702, 1102) in einer versetzten Anordnung, wobei jeder MUT der Mehrzahl von MUTs eine Dimension (714, 1114) entlang einer ersten Richtung (Y) aufweist, die größer als eine Dimension (712, 1112) entlang einer zweiten Richtung (X) ist; und
wobei die versetzte Anordnung eine erste Reihe von MUTs, die entlang der ersten Richtung (Y) ausgerichtet sind, und eine zweite Reihe von MUTs, die entlang der ersten Richtung ausgerichtet sind, aufweist; wobei die zweite Reihe relativ zu der ersten Reihe verschoben ist, um eine Verschiebungsdistanz (VD) (708, 1108) entlang der ersten Richtung zwischen einem MUT in der ersten Reihe (702a, 1102a) und seinem nächsten Nachbar in der zweiten Reihe (702b, 1102b) zu ergeben;
**dadurch gekennzeichnet, dass** eine Projektionsfläche von jedem der Mehrzahl von MUTs eine Form eines Rechtecks (702) oder einer Ellipse (1102) hat.

2. Die Wandleranordnung gemäß Anspruch 1, wobei:
eine Mittelpunkt-zu-Mittelpunkt-Distanz zwischen der Mehrzahl von MUTs in einer Richtung sich von einer Mittelpunkt-zu-Mittelpunkt-Distanz zwischen der Mehrzahl von MUTs in einer anderen Richtung unterscheidet.

3. Die Wandleranordnung gemäß Anspruch 1, wobei jeder MUT der Mehrzahl von MUTs eine längliche Form aufweist, die eine oder mehrere Moden der Vibration in einem biegefähigen Betriebsmodus unterstützt.

4. Die Wandleranordnung gemäß einem der Ansprüche 1 bis 3, wobei jeder MUT (602) der Mehrzahl der MUTs in einem zufälligen Winkel zu einer axialen Richtung gedreht ist, während jeder MUT (602) im Wesentlichen an der ersten Richtung (Y) ausgerichtet ist.

5. Die Wandleranordnung gemäß Anspruch 1, wobei jeder der Mehrzahl von MUTs mindestens eines aus der Übertragung einer Ultraschallwelle und des Empfangens einer Ultraschallwelle durchführt.

6. Die Wadleranordnung gemäß Anspruch 1, wobei jeder der Mehrzahl von MUTs in mindestens einem aus einer symmetrischen Vibrationsmode und einer asymmetrischen Vibrationsmode betrieben wird.

## Revendications

1. Réseau de transducteurs, comprenant :
une pluralité de transducteurs ultrasoniques micro-usinés (MUT) (402, 502, 602, 702, 1102) disposés en quinconce, chaque MUT de la pluralité de MUT ayant une dimension (714, 1114) le long d'une première direction (Y) supérieure à une dimension (712, 1112) le long d'une deuxième direction (X) ; et
dans lequel la disposition en quinconce comprend une première rangée de MUT alignés le long de la première direction (Y) et une deuxième rangée de MUT alignés le long de la première direction ; la deuxième rangée étant décalée par rapport à la première rangée pour donner une distance de décalage (VD) (708, 1108) le long de la première direction entre un MUT dans la première rangée (702a, 1102a) et son plus proche voisin dans la deuxième rangée (702b, 1102b) ;
dans lequel une zone de projection de chacun de la pluralité de MUT présente une forme de rectangle (702) ou d'ellipse (1102).

2. Réseau de transducteurs selon la revendication 1, dans lequel :
une distance centre-à-centre entre la pluralité de MUT dans une direction est différente d'une distance centre-à-centre entre la pluralité de MUT dans une autre direction.

3. Réseau de transducteurs selon la revendication 1,
dans lequel chaque MUT de la pluralité de MUT présente une forme allongée qui supporte un ou plusieurs modes de vibration dans un mode de fonctionnement en flexion.

4. Réseau de transducteurs selon l'une quelconque des revendications 1 à 3,
dans lequel chaque MUT (602) de la pluralité de MUT est tourné d'un angle aléatoire par rapport à une direction axiale, tandis que chaque MUT (602) est sensiblement aligné avec la première direction (Y).

5. Réseau de transducteurs selon la revendication 1,
dans lequel chacune de la pluralité de MUT effectue au moins l'une parmi la transmission d'une onde ultrasonique et la réception d'une onde ultrasonique.

6. Réseau de transducteurs selon la revendication 1,
dans lequel chacun de la pluralité de MUT fonctionne dans au moins un parmi un mode de vibration symétrique et un mode de vibration asymétrique.
